# EUROPÄISCHE PATENTSCHRIFT

(11) **EP 1 728 482 B1**
(45) Veröffentlichungstag und Bekanntmachung des Hinweises auf die Patenterteilung: **29.12.2010**
(21) Anmeldenummer: 05011682.1
(22) Anmeldetag: 31.05.2005
(51) Int. Cl.: A61B 19/00

(54) **Selbsteinstellendes Operationslampensystem**
Selfadjusting surgical lamp system
Système de lampe chirurgicale autoajustable

(43) Veröffentlichungstag der Anmeldung: 06.12.2006
(73) Patentinhaber: BrainLAB AG, 85622 Feldkirchen (DE)
(72) Erfinder: Goldbach, Günter, 85457 Wörth/Wifling (DE); Rossner, Holger-Claus, 85622 Feldkirchen (DE)
(74) Vertreter: Schwabe - Sandmair - Marx

(56) Entgegenhaltungen:
- EP-A- 0 421 130
- EP-A- 1 340 470
- DE-A1- 10 246 147
- US-A- 4 578 575
- US-A- 4 639 838
- US-A- 5 038 261
- US-A- 5 383 105
- US-A1- 2003 153 978
- US-A1- 2003 210 812
- US-B1- 6 434 329

## Beschreibung

Die Erfindung betrifft ein kombiniertes Operationslampen-Bilderfassungssystem sowie ein Verfahren zum Betrieb eines solchen Systems.

Ein gattungsgemäßes System ist aus der EP 0 421 130 A2 bekannt. Es weist eine Operationslampe auf, die an einer beweglichen Halterung angebracht ist, sowie mindestens eine Kamera, die in einem festen oder bekannten bzw. erfassbaren Positionsverhältnis zur Operationslampe angeordnet ist.

Während einer Operation muss darauf geachtet werden, dass das Operationsgebiet ständig möglichst schattenfrei ausgeleuchtet ist. Dies ist mit herkömmlichen Operationslampen manchmal schwierig, wenn die Behandelnden zwischen Lampen und Patient geraten, und in solchen Fällen muss die Operationslampe derzeit immer noch per Hand verstellt bzw. neu angeordnet werden, um wieder eine optimale Ausleuchtung zu erreichen.

Die US-4,639,838 A beschreibt eine schattenlose Beleuchtungsvorrichtung für die medizinischen Verwendung. Die Beleuchtungsvorrichtung schließt eine Leuchtvorrichtung für das emittieren eines Lichtstrahls wie zum Beispiel eines Infrarotlichtstrahls, der einer Frequenzmodulation auf einem Patienten auf einem Operationstisch unterworfen wird, um einen Bestrahlungsbereich darauf auszubilden, eine Bild erzeugende Vorrichtung, die ein Bild des Bestrahlungsbereichs in Übereinstimmung mit einem Lichtstrahl, der von dem bestrahlten Teil des Patienten reflektiert wird, ausbildet und einen Sensor ein, um die Informationen zu vergleichen, die eine Lichtkonzentrationsposition von der Leuchtvorrichtung betrifft. In diesem Vergleich wird, wenn die Bilderzeugungsposition von der Lichtkonzentrationsposition beabstandet ist, ein Ausgang bereitgestellt, der Informationen beinhaltet, die den Positionsunterschied zwischen den Positionen betreffen und, in Erwiderung auf solch einen Ausgang, wird die Leuchtvorrichtung in X-Achsenrichtung, Y-Achsenrichtung und Senkrechtrichtung einer Koordinatenoberfläche gefahren, damit die Lichtkonzentrationsposition dieselbe wird wie Bilderzeugungsposition.

### ZUSAMMENFASSUNG DER ERFINDUNG

Es ist die Aufgabe der vorliegenden Erfindung, ein optimiertes Operationslampen-Bilderfassungssystem sowie ein Verfahren zu dessen Betrieb bereitzustellen. Insbesondere soll die optimale Ausleuchtung des Operationsfeldes ermöglicht werden, ohne dass ständig eine manuelle Nachstellung erfolgen muss.

Diese Aufgabe wird erfindungsgemäß durch die Merkmale der unabhängigen Patentansprüche gelöst. Die Unteransprüche definieren bevorzugte Ausführungsformen der Erfindung.

### VORTEILE DER ERFINDUNG

Vorteilhaft wird ein kombiniertes Operationslampen-Bilderfassungssystem bereitgestellt, bei dem die bewegliche Halterung

Bewegungseinrichtungen aufweist, die auf der Basis von Bildsignalen ansteuerbar sind, welche von der Kamera erfasst werden. Mit anderen Worten wird der Bildinhaft des Kamerabildes sinnvoll mit der Positionierung der Operationslampe verknüpft, und zwar so, dass die Lampe sich selbst bezüglich ihrer Position einstellen kann, um eine schattenfreie und optimale Ausleuchtung des Operationsfeldes zu erreichen. Falls dem Bildinhalt des Kamerabildes zu entnehmen ist, dass die Ausleuchtung nicht mehr optimal ist (wenn beispielsweise Instrumente, die im Operationsfeld vorhanden sein sollten, aus dem Kamerabild verschwinden), kann eine automatische und selbsttätige Nachstellung der Operationslampenposition erfolgen (bis die Instrumente und damit das Operationsfeld wieder im Kamerabild zu sehen sind und entsprechend auch von der Operationslampe beleuchtet werden). Dies ist möglich, weil Kamera und Operationslampe zumindest im Wesentlichen auf dasselbe Gebiet gerichtet sein sollten.

Es sind Datenübertragungseinrichtungen zur Datenübertragung zwischen den Bewegungseinrichtungen und der Kamera vorgesehen. Außerdem umfasst das System eine in die Datenübertragung eingebundene Steuerungs-bzw. Regelungseinheit in Form eines Navigationssystems, insbesondere ein medizintechnisches Navigationssystem, wobei dieses Navigationssystem die Steuerungs- bzw. Regelungseinheit für die Steuerung bzw. Regelung der Bewegungseinrichtungen bildet oder umfasst. Grundsätzlich, nicht als Teil der Erfindung kann aber auch eine separate Steuerungs- bzw. Regelungseinheit vorgesehen, ohne Navigationssystem oder zusätzlich zum Navigationssystem. Die Datenübertragungseinrichtungen sind zum Beispiel kabellose Datenübertragungs-einrichtungen mit Sendern und Empfängern oder Datenübertragungskabel.

Es besteht die Möglichkeit, die Kamera mittig im Operationslampengehäuse anzuordnen, und außerdem ist es möglich, die Kamera mit einem Gehäuse zu versehen, das auch als Schaft für den sterilen Haltegriff der Operationslampe dient.

Weiter vorteilhaft umfasst das vorteilhafte System eine Lichtquelle für Licht in einem abgegrenzten Spektralbereich, insbesondere eine Infrarotlichtquelle. Diese Lichtquelle kann zusammen mit der Kamera ansteuerbar sein oder direkt die Kamera ansteuern, wenn sie selbst eingeschaltet wird. Hierbei ist es insbesondere von Vorteil, wenn die Kamera zur Erfassung des Lichtes in dem vorgenannten Spektralbereich ausgebildet ist, vorzugsweise mit einem dafür durchlässigen Filter versehen ist

Das System weist vorteilhaft mehrere zusammen und individuell oder abwechselnd verwendbare Kameras auf. Dem System ist ausser dem ein durch das Navigationssystem eindeutig identifizierbares, insbesondere dort aufgrund einer charakteristischen Referenzanordnung bekanntes und/oder hinterlegtes, Instrument oder einen Satz solcher Instrumente zugeordnet. Mit solchen "systembekannten" Instrumenten ist es besonders vorteilhaft möglich, entsprechende Referenzanordnungen durch die Kamera zu verfolgen (zu tracken) und auch festzustellen, wenn sie das Kamera-Sichtgebiet und damit auch das Gebiet der optimalen Ausleuchtung verlassen, so dass eine effiziente selbstständige Nachstellung erfolgen kann. Grundsätzlich können Instrumente auch anhand einer Konturerkennung (Instrumenten-Außenkontur) identifiziert werden.

Vorteilhaft wird ein Verfahren zum Betrieb eines kombinierten Operationslampen-Bilderfassungsystems bereit gestellt, welches umfasst die Steuerung bzw. Regelung der beweglichen Halterung mittels ansteuerbarer Bewegungseinrichtungen auf der Basis von Signalen der Kamera, um das Operationslampen-Bilderfassungssystem in eine gewünschte Position zu bringen. Wiederum besteht hier die Möglichkeit, eine in ein Navigationssystem integrierte Steuerungs- bzw. Regelungseinheit zur Verfügung zu stellen und in die Datenübertragung einzubinden. Auch kann gemäß einer Ausführungsform eine Lichtquelle für Licht in einem abgegrenzten Spektralbereich, insbesondere eine Infrarotlichtquelle, zusammen mit der Kamera ansteuerbar sein und angesteuert werden, wobei die Kamera insbesondere zur Erfassung des Lichtes in diesem Spektralbereich ausgebildet, vorzugsweise mit einem dafür durchlässigen Filter versehen ist, und wobei die Operationslampe mit Hilfe der Lichtreflektionen ausgerichtet wird.

Das Operationslampen-Bilderfassungssystem kann nur eine einzige oder mehrere, mindestens jedoch eine, einzeln, zusammen und individuell oder abwechseind verwendbare Kameras aufweisen, wobei bei einer vorteilhaften Verfahrensvariante deren Bildsignale ausgewertet werden, um zu ermitteln, ob das Sichtfeld einer der Kameras eingeschränkt ist, wobei in diesem Fall eine andere Kamera oder mehrere andere Kameras zur Verwendung kommen, deren Sichtfeld nicht eingeschränkt ist. Die Lichtquellen können dabei von bzw. mit den einzelnen Kameras einzeln angesteuert werden, und sie sind insbesondere an individuellen Halterungen (Haltearmen) angeordnet .

Bei der Verwendung mehrerer Kameras ist es möglich, Bildsignale zu vergleichen bzw. abzugleichen und Übereinstimmungsmängel festzustellen, um entsprechende Korrekturmaßnahmen ergreifen zu können.

Weiter Vorteilhaft wird in dem Verfahren die Kamera verwendet, um Instrumente zu tracken, wobei insbesondere ein durch das Navigationssystem eindeutig identifizierbares, insbesondere dort aufgrund einer charakteristischen Referenzanordnung bekanntes oder hinterlegtes, Instrument oder ein Satz solcher Instrumente positionell erfasst wird.

Eine andere Möglichkeit der Bildsignalauswertung besteht darin, auf der Basis der von der Kamera aufgefangenen Bildsignale den Fokus oder das Ausleuchtungsfeld der Operationslampe zu verändern.

Weiter Vorteilhaft wird auch ein kombiniertes Operationslampen-Bilderfassungssystem mit einer Operationslampe bereitgestellt, die an einer Halterung angebracht ist, und mit einer Kamera, die in einem festen oder bekannten bzw. erfassbaren Positionsverhältnis zur Operationslampe angeordnet ist, dadurch gekennzeichnet, dass die Kamera die einzige Kamera des Systems und zur dreidimensionalen Positionserfassung ausgebildet ist. In diesem Umfeld können alle oben dargestellten Systemmerkmale verwirklicht werden.

Vorteilhaft wird auch ein Verfahren zum Betrieb eines kombinierten Operationslampen-Bilderfassungssystems offenbart, mit einer Operationslampe, die an einer Halterung angebracht ist, und mit einer Kamera, die in einem festen oder bekannten bzw. erfassbaren Positionsverhältnis zur Operationslampe angeordnet ist, bei dem mittels einer einzigen Kamera, die zur dreidimensionalen Positionserfassung ausgebildet ist, Positionserfassungen durchgeführt werden. In diesem Umfeld können wiederum alle oben dargestellten Verfahrensmerkmale verwirklicht werden.

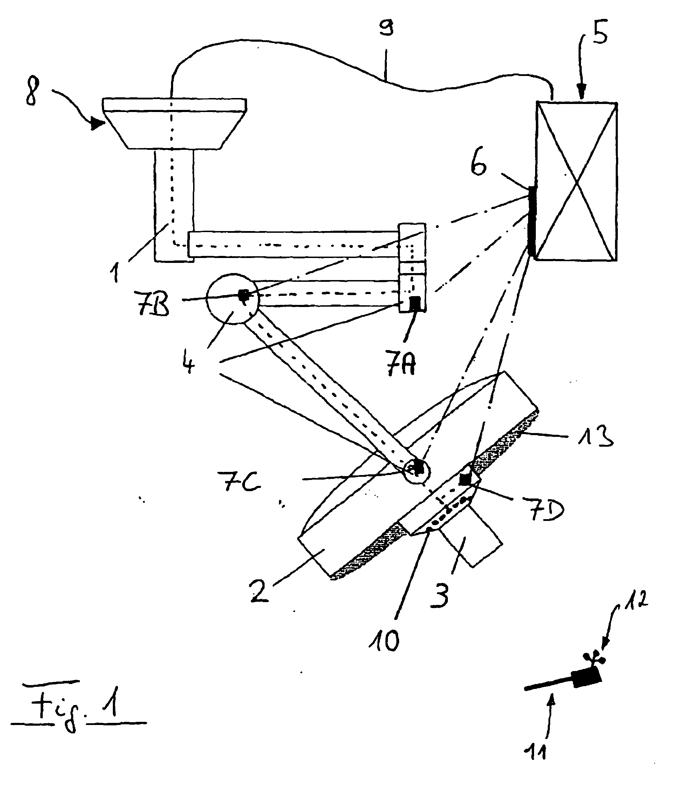

Die Erfindung wird im Weiteren anhand einer Ausführungsform und unter Bezugnahme auf die einzige beiliegende Figur (Figur 1) näher erläutert. Sie kann alle hierin beschriebenen Merkmale einzeln oder in jedweder Kombination umfassen. Die Figur 1 zeigt eine schematische Darstellung eines erfindungsgemäßen kombinierten Operationslampen-Bilderfassungssystems.

In der Figur 1 ist eine Operationslampe 2 gezeigt, die über eine bewegliche Halterung 1 an einer Deckenbefestigung 8 angebracht ist. Die bewegliche Halterung 1 umfasst einige Arme, die gelenkig miteinander verbunden sind; auch die Lampe 2 ist gelenkig am letzten Arm angebracht. In den Gelenken sind Bewegungseinrichtungen vorgesehen, im vorliegenden Fall Drehstellmotoren, und dies sind durch ein einziges Bezugszeichen 4 bezeichnet worden. Mit Hilfe der Drehstellmotoren oder Servos 4 in den Gelenken kann die Lampe 2 an den Armen in jedwede gewünschte Stellung verfahren werden. Die Operationslampe kann in einem oder in mehreren Achsen Bewegungseinrichtungen besitzen. Eventuell kann die Operationslampe nur im letzten kardanisch aufgehängten Glied verstellt werden.

Es ist hier anzumerken, dass es sich nicht unbedingt um Drehstellmotoren handeln muss, alle bekannten Bewegungseinrichtungen sind verwendbar, so auch insbesondere Längsstelleinrichtungen, wie beispielsweise Teleskopiersysteme.

Die Lampe 2 weist Beleuchtungsmittel 13 zur Ausleuchtung des Operationsfeldes auf. Mittig trägt sie eine Kamera 3, die optische Bildsignale erfasst. An der Kamera 2 sind Infrarot-LEDs 10 angeordnet. Es kann aber auch die Lampenbeleuchtung als Navigationslicht verwendet werden. Vor der Kamera ist in Figur 1 noch schematisch ein chirurgisches Instrument 11 gezeigt, das eine Referenzanordnung 12 mit drei Reflektoren für Infrarotlicht trägt. Es können anstatt der Reflektoren auch aktive Elemente (z.B. LEDs) verwendet werden (aktive Emitter-Referenzanordnungen).

In der Figur 1 sind gleichzeitig zwei mögliche Systeme zur Datenübertragung zwischen einer Steuerungs- bzw. Regelungseinheit 5 und den anderen Systemteilen (Drehservos 4, Kamera 3) aufgezeigt. Diese beiden Datenübertragungssysteme müssen nicht nebeneinander existieren, sie können einzeln und unabhängig voneinander verwendet werden. Die Steuerungs- bzw. Regelungseinheit 5 ist grundsätzlich eine integrierte Schaltung oder ein Computer zur Signalverarbeitung. Es kann sich bei ihr um ein medizintechnisches Navigationssystem bzw. ein medizintechnisches Trackingsystem handeln, wie es beispielsweise in der DE 19 639 615 A1 beschrieben wird. Die Einheit 5 kann auch lediglich ein datenverarbeitender Teil dieses Navigationssystems sein.

Eine Art der dargestellten Datenübertragung beruht auf einer Kabelverbindung 9, welche die Einheit 5 mit allen Servo-Stellmotoren 4 sowie mit der Kamera 3 und der LED-Anordnung 10 verbindet.

Die zweite dargestellte Möglichkeit betrifft eine kabellose Datenübertragung, bei der ein Sender/Empfänger 6 an der Steuerungs- bzw. Regelungseinheit 5 angeordnet ist. Dieser Sender/Empfänger 6 kommuniziert mit weiteren Sendern/Empfängern 7A, 7B, 7C (an den Servo-Stellmotoren 4) sowie 7D (für Kamera 3 und Infrarot-LEDs 10).

Insgesamt dienen die Sender/Empfänger 6 bzw. 7 (7A bis 7D) dem Datentausch zwischen der Einheit 5 und den restlichen Systemen. Insbesondere werden von der Einheit 5 Bewegungsbefehle an die Servos 4, sowie Ein- und Ausschaltbefehle an Kamera 3 und LED-Anordnung 10 abgegeben, wobei andererseits die Servos 4 auch Positionsrückmeldungen an die Einheit 5 übermitteln können und die Kamera 3 Bilder zur Verarbeitung an die Einheit 5 transferieren kann.

Im Einsatz wird das erfindungsgemäße System zum Beispiel dazu verwendet, eine schattenfreie Beleuchtung eines Bereichs (Operationsfeld) zu erreichen, wobei die Kamera zweidimensionale oder dreidimensionale Bilder liefert, welche verwendet werden, um die Position der Lampe 2 zu steuern bzw. zu regeln und beispielsweise das chirurgische Instrument 11 in Kombination mit der Einheit 5 zu verfolgen. Dabei macht man sich die Tatsache zu Nutze, dass ein bestimmtes Instrument 11 mit einer eindeutig identifizierbaren Referenzanordnung 12 (dem Navigationssystem 5 bekannt) bei der Operation schon definitionsgemäß im Operationsfeld befindlich sein muss. Wenn nun durch die Infrarot-LEDs 10 intermittierend oder kontinuierlich Licht abgegeben wird, so wird dieses durch die Markeranordnung 12 reflektiert und die Kamera 3 kann diese Reflektion erfassen. Auf der Basis dieses erfassten Bildes kann dann festgestellt werden, ob sich die Operationslampe 2 noch am optimalen Ort befindet bzw. optimal eingestellt ist, weil die entsprechenden Bilddaten an die Steuerungs- bzw. Regelungseinheit 5 (Navigationssystem) übertragen und dort ausgewertet werden. Wenn sich ergibt, dass eine optimale Position der Lampe 2 nicht mehr gegeben ist, kann die Einheit 5 Nachstellbefehle an die Servo-Stellmotoren 4 geben, welche die Lampe 2 dann so verfahren, dass das Instrument wieder optimal aufgenommen wird, was gleichzeitig bedeutet, dass die Lampe 2 optimal und schattenfrei das Operationsfeld ausleuchtet.

Bei einer einfachen Regelung würde beispielsweise festgestellt, dass die Markeranordnung 12 sich immer wieder oder über längere Zeit in einer Richtung aus dem Bild entfernt oder von einer Richtung her immer verdeckt wird. Durch das Kamerabild und dessen Auswertung in der Einheit 5 wäre diese Richtung feststellbar und es könnte eine dem entgegenwirkende, gewünschte Bewegung der Lampe 2 errechnet werden, die dann durch entsprechende Ansteuerung der Servo-Stellmotoren 4 realisiert wird. Die Kamera hat dann wieder eine optimale Sichtlinie, was auch bedeutet, dass das Operationsfeld optimal ausgeleuchtet ist.

Die Kamera kann gleichzeitig verwendet werden, um Instrumente wie das schematisch dargestellte Instrument 11 anhand ihrer Markeranordnungen 12 zu identifizieren und/oder zu tracken, das heißt, positionell zu orten und zu verfolgen. Dies ist mit einer einzigen Kamera möglich, wenn die Instrumente eindeutig identifizierbar und ihre Form bzw. ihre charakteristischen Markeranordnungen in einem Speicher des Navigationssystems 5 hinterlegt sind. Es können aber auch mehrere Kameras zur Verwendung kommen, die neben der Trackingfunktion und der Lampenpositionierung auch redundante Funktionen erfüllen können, um Sichtbehinderungen oder Bildinhaltsdifferenzen zu eliminieren.

## Patentansprüche

1. System mit einer Operationslampe (2), die an einer beweglichen Halterung (1) angebracht ist, wobei die bewegliche Halterung (1) Bewegungseinrichtungen (4) aufweist, die auf der Basis von Signalen ansteuerbar sind,
wobei
das System ein kombiniertes Operationslampen-Bilderfassungssystem ist;
das System mindestens eine Kamera (3), aufweist, die in einem festen oder bekannten bzw. erfassbaren Positionsverhältnis zur Operationslampe (2) angeordnet ist;
die Bewegungseinrichtungen (4) auf der Basis von Bildsignalen angesteuert werden, welche von der Kamera (3) erfasst werder wobei die Operationslampe (2) ein Operationsfeld automatisch ausleuchtet; Datenübertragungseinrichtungen (6, 7, 9) zur Datenübertragung zwischen den Bewegungseinrichtungen (4) und der Kamera (3) vorgesehen sind; das System eine in die Datenübertragung eingebundene Steuerungs- bzw. Regelungseinheit (5) umfasst;
die Steuerungs- bzw. Regelungseinheit (5) ein Navigationssystem (5) insbesondere ein medizintechnisches Navigationssystem (5) ist; und
das System ein durch das Navigationssystem (5) eindeutig identifizierbares, insbesondere dort aufgrund einer charakteristischen Referenzanordnung bekanntes und/oder hinterlegtes, Instrument oder einen Satz solcher Instrumente umfasst.

2. System nach Anspruch 1, **dadurch gekennzeichnet, dass** das Navigationssystem (5) die Steuerungs- bzw. Regelungseinheit für die Steuerung- bzw. Regelung der Bewegungseinrichtungen (4) bildet bzw. umfasst.

3. System nach Anspruch 1 oder 2, **dadurch gekennzeichnet, dass** die Datenübertragungseinrichtungen(6, 7, 9) kabellose Datenübertragungseinrichtungen mit Sendern und Empfängern (6, 7) oder Datenübertragungskabel (9) sind.

4. System nach einem der Ansprüche 1 bis 3, **dadurch gekennzeichnet, dass** die Kamera (3) mittig im Operationslampengehäuse angeordnet ist.

5. System nach einem der Ansprüche 1 bis 4, **dadurch gekennzeichnet, dass** die Kamera (3) ein Gehäuse aufweist, das als Schaft für den sterilen Handgriff der Operatoinslampe (2) dient.

6. System nach einem der Ansprüche 1 bis 5, **dadurch gekennzeichnet, dass** es eine Lichtquelle für Licht in einem abgegrenzten Spektralbereich, insbesondere eine Infrarotlichtquelle (10) umfasst, die zusammen mit der Kamera (3) ansteuerbar ist, wobei die Kamera (3) insbesondere zur Erfassung des Lichtes in diesem Spektralbereich ausgebildet, vorzugsweise mit einem dafür durchlässigen Filter versehen ist.

7. System nach einem der Ansprüche 1 bis 6, **dadurch gekennzeichnet, dass** es mehrere zusammen und individuell oder abwechselnd verwendbare Kameras aufweist.

8. System nach einem der Ansprüche 1 bis 6, **dadurch gekennzeichnet, dass** die Kamera (3) die einzige Kamera des Systems und zur dreidimensionalen Positionserfassung ausgebildet ist.

9. Verfahren zum Betrieb eines kombinierten Operationslampen-Bilderfassungssystems mit einer Operationslampe (2), die an einer beweglichen Halterung (1) angebracht ist, und mit mindestens einer Kamera (3), die in einem festen oder bekannten bzw. erfassbaren Positionsverhältnis zur Operationslampe (2) angeordnet ist, bei dem die bewegliche Halterung (1) mittels ansteuerbarer Bewegungseinrichtungen (4) auf der Basis von Bildsignalen der Kamera (3)
gesteuert bzw. geregelt wird, welche von der Kamera (3) erfasst werden, um das Operationslampen-Bilderfassungssystem in eine gewünschte Position zu bringen, so dass die Operationslampe (2) ein Operationsfeld automatisch ausleuchtet;bei dem eine Datenübertragung zwischen den Bewegungseinrichtungen (4) und der Kamera (3) über eine in die Datenübertragung eingebundene Steuerungs-bzw. Regelungseinheit, (5) stattfindet;
bei dem die Steuerungs- bzw. Regelungseinheit (5) ein Navigationssystem (5) bevorzugt ein medizintechnisches Navigationssystem (5) ist; und
bei dem die Kamera (3) verwendet wird, um Instrumente zu identifizieren und/oder zu tracken, wobei ein durch das Navigationssystem (5) eindeutig identifizierbares, insbesondere dort aufgrund einer charakteristischen Referenzanordnung bekanntes und/oder hinterlegtes, Instrument oder ein Satz solcher Instrumente positionell erfasst wird.

10. Verfahren nach Anspruch 9, bei dem eine Lichtquelle für Licht in einem abgegrenzten Spektralbereich, insbesondere eine Infrarotlichtquelle (10), zusammen mit der Kamera (3) ansteuerbar ist, wobei die Kamera (3) insbesondere zur Erfassung des Lichtes in diesem Spektralbereich ausgebildet, vorzugsweise mit einem dafür durchlässigen Filter versehen ist, und wobei die Operationslampe (2) mit Hilfe der Lichtreflexionen ausgerichtet wird.

11. Verfahren nach Anspruch 9 oder 10, bei dem das Operationslampen-Bilderfassungssystem mehrere, mindestens zwei, zusammen und individuell oder abwechselnd verwendbare Kameras aufweist, deren Bildsignale ausgewertet werden, um zu ermitteln, ob das Sichtfeld einer der Kameras eingeschränkt ist, wobei in diesem Fall eine andere Kamera oder mehrere andere Kameras zur Verwendung kommen, deren Sichtfeld nicht eingeschränkt ist.

12. Verfahren nach Anspruch 11, bei dem Lichtquellen, die von bzw. mit den einzelnen Kameras einzeln angesteuert werden können, an individuellen Halterungen, insbesondere Haltearmen angeordnet sind.

13. Verfahren nach Anspruch 11 oder 12, bei dem die Kameras verwendet werden, um die Bildsignale abzugleichen und Übereinstimmungsmängel festzustellen.

14. Verfahren nach einem der Ansprüche 9 bis 13, bei dem auf der Basis der von der Kamera (3) aufgefangenen Bildsignale der Fokus oder das Ausleuchtungsfeld der Operationslampe (2) verändert werden.

15. Verfahren nach Anspruch 9 oder 10, wobei mittels einer einzigen Kamera (3), die zur dreidimensionalen Positionserfassung ausgebildet ist, Positionserfassungen durchgeführt werden.

## Claims

1. A system comprising an operation lamp (2) which is attached to a movable mount (1), wherein the movable mount (1) comprises movement devices (4) which can be controlled on the basis of signals, wherein:
the system is a combined operation lamp image detecting system;
the system comprises at least one camera (3) which is arranged in a fixed or known and/or
detectable positional relationship relative to the operation lamp (2);
the movement devices (4) are controlled on the basis of image signals detected by the camera (3),
wherein the operation lamp (2) automatically illuminates an operation field;
data transfer devices (6, 7, 9) are provided for transferring data between the movement devices (4) and the camera (3);
the system includes a control/regulating unit (5) integrated into the data transfer;
the control/regulating unit (5) is a navigation system (5), in particular a medical navigation system (5); and
the system comprises an instrument or set of instruments which are clearly identifiable by the navigation system (5), in particular known to and/or stored in the navigation system (5) on the basis of a characteristic reference array.

2. The system according to claim 1, **characterised in that** the navigation system (5) forms or includes the control/regulating unit for controlling/regulating the movement devices (4).

3. The system according to claim 1 or 2, **characterised in that** the data transfer devices (6, 7, 9) are cable-free data transfer devices with transmitters and receivers (6, 7) or data transfer cables (9).

4. The system according to any one of claims 1 to 3, **characterised in that** the camera (3) is arranged centrally in the operation lamp housing.

5. The system according to any one of claims 1 to 4, **characterised in that** the camera (3) comprises a housing which serves as a shaft for the sterile holding grip of the operation lamp (2).

6. The system according to any one of claims 1 to 5, **characterised in that** it includes a light source for light in a delimited spectral range, in particular an infrared light source (10), which can be controlled together with the camera (3), wherein the camera (3) is in particular designed for detecting light in said spectral range and is preferably provided with a filter which is permeable to light in said range.

7. The system according to any one of claims 1 to 6, **characterised in that** it comprises a number of cameras which can be used together and individually or alternately.

8. The system according to any one of claims 1 to 6, **characterised in that** the camera (3) is the only camera in the system and is designed for detecting positions three-dimensionally.

9. A method for operating a combined operation lamp image detecting system comprising an operation lamp (2) which is attached to a movable mount (1) and comprising at least one camera (3) which is arranged in a fixed or known and/or detectable positional relationship relative to the operation lamp (2), wherein the moveable mount (1) is controlled/regulated by means of controllable movement devices (4) on the basis of image signals from the camera (3), which are detected by the camera (3) in order to move the operation lamp image detecting system to a desired position, such that the operation lamp (2) automatically illuminates an operation field, wherein:
data are transferred between the movement devices (4) and the camera (3) via a control/regulating unit (5) integrated into the data transfer;
the control/regulating unit (5) is a navigation system (5), preferably a medical navigation system (5); and
the camera (3) is used to identify and/or track instruments, wherein an instrument or set of instruments which are clearly identifiable by the navigation system (5), in particular known to and/or stored in the navigation system (5) on the basis of a characteristic reference array, is positionally detected.

10. The method according to claim 9, wherein a light source for light in a delimited spectral range, in particular an infrared light source (10), is controllable together with the camera (3), wherein the camera (3) is in particular designed for detecting light in this spectral range and is preferably provided with a filter which is permeable to light in said range, and wherein the operation lamp (2) is aligned with the aid of the light reflections.

11. The method according to claim 9 or 10, wherein the operation lamp image detecting system comprises a number of cameras - at least two - which can be used together and individually or alternately, wherein image signals from the cameras are evaluated in order to establish whether the field of view of one of the cameras is restricted, wherein in this case, another camera or a number of other cameras whose field of view is not restricted are used.

12. The method according to claim 11, wherein light sources which can be controlled individually by or using the individual cameras are arranged on individual mounts, in particular holding arms.

13. The method according to claim 11 or 12, wherein the cameras are used to match the image signals and to determine mismatches.

14. The method according to any one of claims 9 to 13, wherein the focus or illumination field of the operation lamp (2) is changed on the basis of the image signals captured by the camera (3).

15. The method according to claim 9 or 10, wherein positions are detected by means of a single camera (3) designed for detecting positions three-dimensionally.

## Revendications

1. Système comportant une lampe chirurgicale(2) qui est montée sur un support mobile (1), le support mobile (1) comportant des dispositifs de déplacement (4) qui peuvent être commandés sur la base de signaux, dans lequel
le système est un système combiné de détection d'image et de lampe chirurgicale,
le système comporte au moins une caméra (3) qui est agencée dans une relation de position fixe, connue ou détectable par rapport à la lampe chirurgicale (2),
les dispositifs de déplacement (4) sont commandés sur la base de signaux d'image qui sont détectés par la caméra (3), la lampe chirurgicale (2) éclairant automatiquement un champ opératoire,
des dispositifs de transmission de données (6, 7, 9) sont prévus pour transmettre des données entre les dispositifs de déplacement (4) et la caméra (3),
le système comporte une unité de commande ou de régulation (5) associée à la transmission de données,
l'unité de commande ou de régulation (5) est un système de navigation (5), en particulier un système de navigation médico-technique (5), et
le système comporte un instrument ou un ensemble d'instruments de ce type pouvant être identifié sans équivoque par le système de navigation (5) sur la base d'un réseau de référence caractéristique, ledit instrument ou ensemble d'instrument étant en particulier connu du système de navigation (5) et/ou contenu dans celui-ci.

2. Système selon la revendication 1, **caractérisé en ce que** le système de navigation (5) forme ou inclut l'unité de commande ou de régulation pour commander ou réguler les dispositifs de déplacement (4).

3. Système selon la revendication 1 ou 2, **caractérisé en ce que** les dispositifs de transmission de données (6, 7, 9) sont des dispositifs de transmission de données sans fil comportant des émetteurs et des récepteurs (6, 7) ou des câbles de transmission de données (9).

4. Système selon l'une quelconque des revendications 1 à 3, **caractérisé en ce que** la caméra (3) est agencée au centre du boîtier de lampe chirurgicale.

5. Système selon l'une quelconque des revendications 1 à 4, **caractérisé en ce que** la caméra (3) comporte un boîtier qui sert de poignée pour la prise stérile de la lampe chirurgicale (2).

6. Système selon l'une quelconque des revendications 1 à 5, **caractérisé en ce qu'**il comporte une source de lumière, en particulier une source de lumière infrarouge (10), pour émettre de la lumière dans un domaine spectral délimité laquelle source peut être commandée conjointement avec la caméra (3), dans lequel la caméra (3) est de préférence munie d'un filtre transparent à la lumière, en particulier conçu pour détecter la lumière émise dans ce domaine spectral.

7. Système selon l'une quelconque des revendications 1 à 6, **caractérisé en ce qu'**il comporte plusieurs caméras pouvant être utilisées collectivement et individuellement ou alternativement.

8. Système selon l'une quelconque des revendications 1 à 6, **caractérisé en ce que** la caméra (3) est l'unique caméra du système et est conçue pour la détection de position tridimensionnelle.

9. Procédé pour manoeuvrer un système combiné de détection d'image et de lampe d'opération, comportant une lampe chirurgicale (2) qui est montée sur un support mobile (1), et au moins une caméra (3) qui est agencée dans une relation de position fixe, connue ou détectable par rapport à la lampe chirurgicale (2), dans lequel le support mobile (1) est commandé ou régulé au moyen de dispositifs de déplacement (4) pouvant être commandés sur la base de signaux d'image de la caméra (3), lesdits signaux étant détectés par la caméra (3) pour amener le système de détection d'image et de lampe chirurgicale dans une position voulue de telle sorte que la lampe chirurgicale (2) éclaire automatiquement un champ chirurgicale, dans lequel une transmission de données entre les dispositifs de déplacement (4) et la caméra (3) a lieu par l'intermédiaire d'une unité de commande ou de régulation (5) associée à la transmission de données,
dans lequel l'unité de commande ou de régulation (5) est un système de navigation (5) , de préférence un système de navigation médico-technique (5), et
dans lequel la caméra (3) est utilisée afin d'identifier et/ou de suivre des instruments, la position étant détectée par un instrument ou un ensemble d'instruments de ce type pouvant être identifié sans équivoque par le système de navigation (5) sur la base d'un réseau de référence caractéristique, ledit instrument ou ensemble d'instrument étant en particulier connu du système de navigation (5) et/ou contenu dans celui-ci.

10. Procédé selon la revendication 9, dans lequel une source de lumière, en particulier une source de lumière infrarouge (10), pour émettre de la lumière dans un domaine spectral délimité peut être commandée conjointement avec la caméra (3), dans lequel la caméra (3) est de préférence munie d'un filtre transparent à la lumière, en particulier conçu pour détecter la lumière émise dans ce domaine spectral, et dans lequel la lampe chirurgicale (2) est orientée à l'aide des réflexions de lumière.

11. Procédé selon la revendication 9 ou 10, dans lequel le système de détection d'image et de lampe chirurgicale comporte plusieurs, au moins deux, caméras pouvant être utilisées collectivement et individuellement ou alternativement, dont les signaux d'image sont évalués afin de déterminer si le champ de vision de l'une des caméras est restreint, auquel cas une autre caméra ou plusieurs autres caméras dont le champ de vision n'est pas restreint, sont utilisées.

12. Procédé selon la revendication 11, dans lequel des sources de lumière qui peuvent être commandées individuellement par ou avec les caméras individuelles, sont agencées sur des supports individuels, en particulier des bras de support.

13. Procédé selon la revendication 11 ou 12, dans lequel les caméras sont utilisées pour synchroniser les signaux d'image et déterminer les disconcordances.

14. Procédé selon l'une quelconque des revendications 9 à 13, dans lequel le foyer ou le champ d'éclairage de la lampe chirurgicale (2) est modifié sur la base des signaux d'image capturés par la caméra (3).

15. Procédé selon la revendication 9 ou 10, dans lequel des détections de position sont effectuées au moyen d'une caméra unique (3) configurée pour la détection de position tridimensionnelle.
